# EUROPÄISCHE PATENTSCHRIFT

(11) **EP 2 961 367 B1**
(45) Veröffentlichungstag und Bekanntmachung des Hinweises auf die Patenterteilung: **17.10.2018**
(21) Anmeldenummer: 14707977.6
(22) Anmeldetag: 26.02.2014
(51) Int. Cl.: A61F 13/56, A61F 13/58, A61F 13/62, A44B 18/00, A61F 13/15, B32B 37/00, B32B 37/12, B32B 37/18, B32B 38/00

(54) **WINDELVERSCHLUSSBAND SOWIE VERFAHREN UND VORRICHTUNG ZU DESSEN HERSTELLUNG**
DIAPER CLOSING STRIP AND METHOD AND DEVICE FOR THE PRODUCTION THEREOF
BANDE DE FERMETURE DE COUCHE AINSI QUE PROCÉDÉ ET DISPOSITIF POUR LA FABRIQUER

(30) Priorität: 26.02.2013 DE 102013101886
(43) Veröffentlichungstag der Anmeldung: 06.01.2016
(73) Patentinhaber: Lohmann-koester GmbH & Co. KG, 96146 Altendorf (DE)
(72) Erfinder: KOLIC, Ivica, 91336 Heroldsbach (DE); GRÜNER, Tobias, 91338 Igensdorf (DE); HARTUNG, Florian, 91052 Erlangen (DE)
(74) Vertreter: Molnia, David
(86) Internationale Anmeldenummer: PCT/EP2014/053733
(87) Internationale Veröffentlichungsnummer: WO 2014/131798

(56) Entgegenhaltungen:
- EP-A1- 1 484 041
- EP-A1- 2 145 555
- DE-A1-102007 036 596
- DE-U1-202004 021 382
- US-A1- 2004 236 303
- US-A1- 2011 313 389

## Beschreibung

Die Erfindung betrifft ein Windelverschlussband sowie ein Verfahren und eine Vorrichtung zu dessen Herstellung. Genauer betrifft die Erfindung ein Windelverschlussband, das eine Mehrzahl von mechanischen Verschlusselementen aufweist, sowie ein Verfahren und eine Vorrichtung zu dessen Herstellung.

Windelverschlussbänder zum Verschließen von Windeln wie beispielsweise Baby- und Inkontinenzwindeln sind unter anderem aus der US 4,585,450, der US 5,624,429, der US 6,142,986 und der US 4,043,340 bekannt. Üblicherweise ist eine Windel mit zwei Windelverschlussbändern ausgestattet, wobei jedes Windelverschlussband jeweils an einem der Windelohren im hinteren Taillenbereich der Windel permanent befestigt ist. Beim Anlegen der Windel an einen Windelträger werden die beiden Windelverschlussbänder um die Taille des Windelträgers herum zum vorderen Taillenbereich der Windel geführt und dort mit der Windel in eine lösbare Halteverbindung gebracht. Diese lösbare Halteverbindung kann gemäß dem Stand der Technik sowohl durch eine Klebewirkung als auch auf mechanische Weise oder eine Kombination von beidem erzielt werden. So schlagen beispielsweise die EP1 635 752 A1, die EP0 321 232 A1 und die EP0 755 665 A1 ein Windelverschlussband vor, bei dem ein oder mehrere mechanische Verschlusselemente auf einem Klebestreifen derart angeordnet sind, dass freiliegende Bereiche des Klebestreifens zur Haltewirkung beitragen können.

Ein Windelverschlussband muss dabei zwei gegenläufige Bedingungen erfüllen. Einerseits muss es mit dem vorderen Taillenbereich der Windel eine lösbare Halteverbindung herstellen, die eine ausreichende Haltekraft besitzt, damit sich der Windelverschluss nicht selbstständig öffnet. Ein solches Öffnen würde nicht nur einen korrekten Sitz der Windel gefährden, es birgt darüber hinaus die Gefahr, dass bereits in der Windel befindliche Inhalte auszutreten können. Andererseits muss die lösbare Verbindung schwach genug sein, damit ein Öffnen und anschließendes Wiederverschließen zur Kontrolle der Windel auf Inhalt bzw. zur Anpassung des Sitzes der Windel möglich ist. Eine zu starke Halteverbindung des Windelverschlussbands könnte hier den in Eingriff genommenen Bereich des vorderen Taillenbereichs der Windel, d.h. die Landing-Zone, beschädigen, was einerseits die Funktionstüchtigkeit der Landing-Zone durch herausgerissenes Material einschränken kann und andererseits die klebenden oder mechanischen Verschlusselemente des Windelverschlussbands durch anhaftende herausgerissene Materialreste blockieren kann.

Erschwert wird das Erzielen einer geeigneten Halteverbindung zwischen Windelverschlussband und Landing-Zone besonders dadurch, dass beide Elemente üblicherweise nicht plan zueinander ausgerichtet sind. Stattdessen befinden sie sich an einer Position, die bei angelegter Windel eine gewisse Krümmung tolerieren muss, nämlich die Rundung der Taille bzw. des Rumpfes des Windelträgers. Diese Krümmung ihrerseits ist wiederum nicht konstant sondern variiert mit jeder Körperbewegung des Windelträgers, was eine zusätzliche Beanspruchung der Halteverbindung mit sich bringt.

Weiterhin beansprucht wird die Halteverbindung zwischen den mechanischen Verschlusselementen und der Landing-Zone durch Zugkräfte. Solche Zugkräfte wirken vorwiegend zwischen dem hinteren Taillenbereich der Windel und dem vorderen Taillenbereich der Windel und damit entlang der Erstreckung des Windelverschlussbands. Sie werden unter anderem bewirkt durch Bewegungen des Windelträgers, allen voran durch Veränderungen der Körperhaltung, die den Bauch- bzw. Taillenumfang des Windelträgers vergrößern, wie beispielsweise Sitzen, Hocken oder Vornüberbeugen.

Der Erfindung liegt daher die Aufgabe zu Grunde, ein Windelverschlussband bereitzustellen, das eine ausgewogene Haltewirkung gegenüber der Landing-Zone entfalten kann, die insbesondere auch bei einer statischen oder dynamischen Verformung des Windelverschlussbands oder der Landing-Zone gewährleistet ist, und die eine besondere Widerstandsfähigkeit gegenüber Zugkräften bereitstellen kann, die entlang der Erstreckung des Windelverschlussbands wirken. Ebenfalls Aufgabe der Erfindung ist es, ein Verfahren und eine Vorrichtung zur Herstellung eines solchen Windelverschlussbands bereitzustellen.

Gelöst wird die Aufgabe durch ein Windelverschlussband sowie ein Verfahren und eine Vorrichtung zu dessen Herstellung gemäß den unabhängigen Ansprüchen, wobei die abhängigen Ansprüche jeweils bevorzugte Ausführungsformen verdeutlichen.

Demgemäß schlägt die Erfindung ein Windelverschlussband vor, das ein Substrat umfasst sowie eine darauf angeordnete Mehrzahl von mechanischen Verschlusselementen. Jedes der mechanischen Verschlusselemente umfasst eine vorderseitige Eingriffsfläche und eine rückseitige Befestigungsfläche und ist mit dem Substrat dadurch verbunden, dass zwischen seiner rückseitigen Befestigungsfläche und dem Substrat ein Klebstoffelement angeordnet ist, das die rückseitige Befestigungsfläche des mechanischen Verschlusselements nicht vollständig bedeckt. Das Klebstoffelement weist also eine räumliche Ausdehnung in der Ebene des Substrats auf, die geringer ist als die räumliche Ausdehnung des jeweiligen mechanischen Verschlusselements in der Ebene des Substrats. Das heißt, in einer Draufsicht auf das Substrat wird das Klebstoffelement von dem daran befestigten mechanischen Verschlusselement vollständig verdeckt, wobei das mechanische Verschlusselement das Klebstoffelement an mindestens einer Begrenzung überragt.

Ebenfalls vorgeschlagen wird ein Verfahren zur Herstellung eines Windelverschlussbands, gemäss Anspruch 9, das folgende Schritte umfasst: Bereitstellen eines Substrats; Bereitstellen einer Mehrzahl von mechanischen Verschlusselementen mit jeweils einem Klebstoffelement auf einer rückseitigen Befestigungsfläche; sowie Verbinden der mechanischen Verschlusselemente mit dem Substrat mittels des Klebstoffelements. Dabei wird das jeweilige Klebstoffelement derart bereitgestellt, dass es die rückseitige Befestigungsfläche des mechanischen Verschlusselements nicht vollständig bedeckt.

Des Weiteren schlägt die Erfindung eine Vorrichtung zur Herstellung eines Windelverschlussbands gemäss Anspruch 12 vor, umfassend eine Substratzuführung zur Bereitstellung eines Substrats; Verschlusselementzuführung zur Bereitstellung zumindest eines mechanischen Verschlusselements mit einer vorderseitigen Eingriffsfläche und einer rückseitigen Befestigungsfläche; eine Beschichtungsvorrichtung zum Aufbringen von Klebstoff auf die rückseitige Befestigungsfläche des zumindest einen mechanischen Verschlusselements; und eine Kontaktvorrichtung zum Verbinden des zumindest einen mechanischen Verschlusselements mit dem Substrat mittels des Klebstoffs. Die Beschichtungsvorrichtung umfasst dabei eine Düsenanordnung mit einer Mehrzahl von Düsenöffnungen zum Bereitstellen einer korrespondierenden Mehrzahl von Klebstoffelementen.

Im Allgemeinen kann das Windelverschlussband einen Befestigungsbereich zur permanenten Befestigung an der Windel, insbesondere an den Windelohren, sowie einen Nutzerbereich aufweisen. Der Nutzerbereich ist der Bereich, der beim Anlegen der Windel um die Taille des Windelträgers herum zum vorderen Taillenbereich der Windel geführt wird und dort mit der Landing-Zone der Windel in eine lösbare Halteverbindung gebracht wird. Er umfasst insbesondere den Bereich des Windelverschlussbands, auf dem die Mehrzahl der mechanischen Verschlusselemente angebracht ist. Zwischen dem Befestigungsbereich und dem Nutzerbereich kann ein Zwischenbereich vorgesehen sein, der eine freie Beweglichkeit des Nutzerbereichs gewährleistet und optional dem Windelverschlussband elastische, insbesondere dehnelastische Eigenschaften verleiht. Der Nutzerbereich wiederum kann an einer seiner äußeren Begrenzungen, insbesondere der dem Befestigungsbereich gegenüberliegenden Begrenzung, einen Griffbereich aufweisen, der seinerseits unterschiedliche Eigenschaften haben kann und vornehmlich der Handhabung des Windelverschlussbands beim Anlegen und Verschließen der Windel dienen kann.

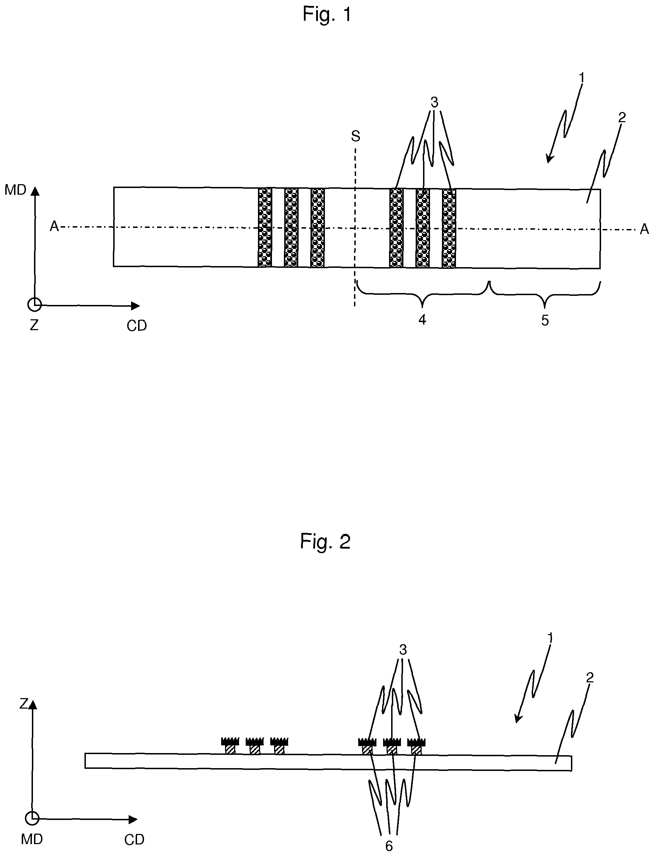

Das Substrat erstreckt sich vorzugsweise über den gesamten Bereich des Windelverschlussbands, insbesondere über den Befestigungsbereich und den Nutzerbereich. Das Substrat ist ein im Wesentlichen flaches Gebilde mit einer Ausdehnung in Maschinenrichtung (Machine-Direction, MD) und Querrichtung (Cross-Direction, CD). Die Ebene des Substrats wird insofern definiert durch die Achsen MD und CD (vgl. Fig. 1 und 2), während sich die Dicke des Substrats senkrecht zur Substratebene in Richtung der Achse Z erstreckt. Unter der Ebene des Substrats ist hierin also die Ebene zu verstehen, in der sich das Substrat in unverformtem Zustand entlang MD und CD erstreckt.

Der Begriff "Maschinenrichtung" bzw. "Machine-Direction" (MD) bezeichnet dabei die Richtung des fortlaufenden Materials des Substrats während des Herstellungsverfahrens. Der Begriff "Querrichtung" bzw. "Cross Direction" (CD) bezeichnet die Richtung, die im Wesentlichen quer zur Maschinenrichtung verläuft.

Das Substrat kann aus einer Vielzahl von Materialien oder Aufbauten ausgebildet sein, wie beispielsweise Papier, Folie, befilmtem oder unbefilmtem Vlies oder Vlies-Folien-Laminat. Diese Materialien sind günstig in der Anschaffung, gut zu verarbeiten und weisen vorteilhafte Eigenschaften auf, wie beispielsweise eine gute Verformbarkeit aus der Ebene des Substrats heraus. Unter einem befilmten Vlies ist dabei ein Vlies zu verstehen, bei dem einseitig ein Kunststofffilm aufgebracht wurde. Als Befilmungsmaterialen werden üblicherweise PE-, PP- oder PET-Polymere verwendet. Die Beschichtungsgrammatur liegt üblicherweise zwischen 5 und 40 g/m². Geeignete Vliese bzw. Vliesstoffe umfassen sowohl Kardenvliese als auch Spinnvliese, wie beispielsweise beschrieben in Albrecht, Fuchs, Kittelmann: Nonwoven Fabrics: Raw Materials, Manufacture, Applications, Characteristics, Testing Processes, Wiley-VCH (2002). Ebenfalls geeignet sind elastische Vliese, insbesondere in sich elastische Vliese und elastische Vlies-Folien-Laminate. Materialien können ferner einlagige oder mehrlagige Filme, coextrudierte Filme, lateral laminierte Filme oder Filme mit Schaumkunststoffschichten umfassen. Die Lagen von solchen Filmen können ihrerseits aus verschiedenen Materialien ausgebildet sein wie beispielsweise Polypropylen, Polyvinylchlorid, Polyethylenterephthalat, Polyethylen, Polyolefin-Copolymeren oder Blends von Polyolefinen wie beispielsweise ein Blend von Polypropylen, LPDE (Low Density Polyethylene) und/oder LLDPE (Linear Low Density Polyethylene), Textilien, geschäumten Materialien und Vliesstoffen.

Die Dicke des Substrats, d.h. seine räumliche Erstreckung in Richtung der Z-Achse, ist vorzugsweise zwischen 20 und 1000 µm und besonders bevorzugt zwischen 50 und 200 µm.

Das Substrat kann in mehrere Zonen unterteilt sein, wobei jede Zone einen anderen Abschnitt des Substrats in CD bezeichnet, der seinerseits einen im Wesentlichen gleichförmigen Aufbau und/oder gleichförmige Eigenschaften aufweist. Die verschiedenen Zonen können aus verschiedenen Materialien ausgebildet sein, die beispielsweise mittels Klebstoff wie selbstklebendem Klebstoff, Ultraschallverbinden, thermischem Verbinden, mechanischem Verbinden, Heften oder einer Kombination davon verbunden sein können. Verschiedene Zonen können auch durch Aktivieren des Materials in einzelnen Bereichen erzeugt werden, beispielsweise durch mechanische, thermische, elektrische und/oder chemische Behandlung, um dem Substratmaterial unterschiedliche Eigenschaften oder Funktionalitäten zu verleihen.

Bevorzugt sind eine oder mehrere Zonen des Substrats in der Ebene des Substrats und insbesondere in CD nicht verformbar, insbesondere nicht dehnbar oder elastisch dehnbar, so dass eine in der Substratebene und insbesondere in CD wirkende Kraft die Erstreckung der betreffenden Zone(n) des Substrats in der Substratebene nicht oder nur unwesentlich verändert. Beispielsweise kann das Substrat in einer oder mehreren Zonen eine Zugfestigkeit gemäß DIN EN 29073-3 von mehr als 10 N/50mm, vorzugsweise zwischen 15 und 200 N/50mm, insbesondere zwischen 20 und 150 N/50mm aufweisen.

Vorzugsweise weist das Substrat derartige Eigenschaften in der Zone, die dem Nutzerbereich des Windelverschlussbands entspricht, und insbesondere in der Zone, in der die Mehrzahl von mechanischen Verschlusselementen angeordnet ist, auf. Die relative Position der mechanischen Verschlusselemente zueinander ist damit feststehend, d.h., der Abstand zwischen den mechanischen Verschlusselementen, insbesondere in CD, variiert nicht bzw. nur unwesentlich. Damit entfaltet die Haltewirkung des Windelverschlussbands gegenüber der Landing-Zone eine besondere Widerstandsfähigkeit gegenüber Zugkräften, die entlang der Erstreckung des Windelverschlussbands, insbesondere in CD, wirken. Eine mögliche Erklärung dafür ist, dass sich aufgrund der beschriebenen Ausgestaltung die genannten Zugkräfte gleichmäßig auf sämtliche mechanischen Verschlusselemente verteilen, so dass sich die Haltekräfte der einzelnen mechanischen Verschlusselemente zu einer Gesamthaltekraft addieren, die deutlich größer ist als die Haltekraft eines einzelnen mechanischen Verschlusselements. Idealerweise entspricht die Gesamthaltekraft der Summe der Haltekräfte der einzelnen mechanischen Verschlusselemente. Ist das Substrat hingegen zwischen den mechanischen Verschlusselementen in der Ebene des Substrats und insbesondere in CD dehnbar, so könnte die Zugkraft in CD auf das in Zugrichtung vorderste mechanische Verschlusselement im Sinne einer Scherkraft einwirken, ohne an das dahinterliegende weitere mechanische Verschlusselement bzw. an die dahinterliegenden weiteren mechanischen Verschlusselemente übertragen zu werden. Eine mögliche Folge wäre, dass die Zugkraft in CD die mechanischen Verschlusselemente nacheinander einzeln von der Landing-Zone löst, womit die Gesamthaltekraft in CD lediglich der Haltekraft des jeweils in Zugrichtung vordersten mechanischen Verschlusselements entspräche. Die genannte bevorzugte Ausgestaltung trägt insoweit zur Verbesserung der lösbaren Halteverbindung zwischen Windelverschlussband und Landing-Zone bei.

Bevorzugt sind eine oder mehrere Zonen des Substrats in eine Richtung aus der Substratebene heraus verformbar, insbesondere elastisch verformbar. Bevorzugt lässt sich das Substrat also in den betreffenden Zonen in Richtung der Z-Achse biegen, knicken und/oder krümmen was insbesondere auch ein Aufrollen des Substrats umfasst.

Besonders bevorzugt weist das Substrat in der Zone, die dem Nutzerbereich des Windelverschlussbands entspricht, und insbesondere in der Zone, in der die Mehrzahl von mechanischen Verschlusselementen angeordnet ist, eine gute Verformbarkeit aus der Substratebene heraus bzw. eine gute Verformbarkeit in Richtung der Z-Achse auf, die es insbesondere gestattet, dass das Substrat in diesem Bereich gebogen, geknickt, gekrümmt oder gerollt werden kann. Diese Verformbarkeit ermöglicht eine Anpassung des Windelverschlussbands an die Form der Landing-Zone bzw. an die Körperform des Windelträgers. Besonders hervorzuheben ist dabei die resultierende dynamische Anpassungsfähigkeit des Windelverschlussbands an Formveränderungen des Windelverschlussbands und/oder der Landing-Zone, die durch Bewegungen des Windelträgers hervorgerufen werden. Auch dies trägt wesentlich zur Verbesserung der lösbaren Halteverbindung zwischen Windelverschlussband und Landing-Zone bei.

Auf dem Substrat angeordnet ist eine Mehrzahl von mechanischen Verschlusselementen. Mehrzahl bedeutet in diesem Zusammenhang zwei oder mehr, also beispielsweise 2, 3, 4, 5, 6 oder 7. Definitionsgemäß sind die Verschlusselemente in dem Bereich des Substrats angeordnet, der dem Nutzerbereich entspricht. Der Nutzerbereich kann sich indes weiter erstrecken als der Bereich, in dem die Verschlusselemente angeordnet sind. Er kann insbesondere weitere Elemente oder Bereiche aufweisen, die zur lösbaren Halteverbindung beitragen, wie beispielsweise selbstklebende Elemente, wie sie unter anderem offenbart sind in der US 4,710,536, der US 5,019,071 oder der US 3,932,328.

Jedes der mechanischen Verschlusselemente umfasst eine vorderseitige Eingriffsfläche und eine rückseitige Befestigungsfläche. Die vorderseitige Eingriffsfläche wird durch eine Mehrzahl von männlichen Befestigungselementen gebildet, die sich ausgehend von einer Trägerlage erstrecken. Die Rückseite der Trägerlage wiederum, d.h., die Fläche der Trägerlage, die der Fläche, von der aus sich die männlichen Befestigungselemente erstrecken, gegenüberliegt, bildet die rückseitige Befestigungsfläche.

Die männlichen Befestigungselemente sind geeignet, faserförmige Materialien mit einer Mehrzahl von komplementären weiblichen Befestigungselementen einzugreifen. Jedes männliche Befestigungselement umfasst üblicherweise einen Schaft, der an seinem einen Ende mit der Trägerlage des mechanischen Verschlusselements verbunden ist und der an seinem anderen Ende einen verbreiterten oder gekrümmten Abschnitt aufweist. Alternativ kann der Schaft anstatt des verbreiterten Abschnitts eine konische, zylindrische, kugel-, halbkugel- oder pyramidenförmige Form aufweisen.

Der verbreiterte Abschnitt eines männlichen Befestigungselements kann verschiedene Formen aufweisen, wie beispielsweise die Form eines Hakens, eines T, eines J, eines Pilzkopfs einschließlich konkav geformter Köpfe oder scheibenförmiger Köpfe, oder jede beliebige andere Form, die einen Eingriff mit komplementären weiblichen Befestigungselementen gestattet.

Die männlichen Befestigungselemente können aus einer Vielzahl von Materialien hergestellt sein, einschließlich thermoplastischer Polymere wie beispielsweise Nylon, Polyester, Polyolefinen oder einen Kombination dieser. Die männlichen Befestigungselemente umfassen jedoch vorzugsweise das Material, aus dem auch die Trägerlage des mechanischen Verschlusselements gebildet ist.

Die Abmessungen eines einzelnen männlichen Befestigungselements können in Abhängigkeit vom Verwendungszweck und von der Struktur und der Beschaffenheit der komplementären weiblichen Befestigungselemente variieren. Bevorzugt hat ein männliches Befestigungselement einen Schaft mit einem Durchmesser zwischen 10 µm und 250 µm und eine Länge zwischen 40 µm und 2 mm. Der verbreiterte Bereich am Ende des Schafts gegenüber der Trägerlage weist dabei bevorzugt einen Durchmesser auf, der das 1,5-fache bis 5,0-fache des Durchmessers des Schafts beträgt.

Vorzugsweise sind die männlichen Befestigungselemente mit der Trägerlage des mechanischen Verschlusselements integral ausgebildet. Es ist jedoch auch möglich, dass die männlichen Befestigungselemente einzeln oder als Gruppen mit einer gemeinsamen Basis an der Trägerlage angebracht sind. So können einzelne Haken oder Gruppen von Haken mit der Trägerlage mittels Klebstoff, Ultraschallbinden oder thermischen Binden verbunden sein.

Üblicherweise sind auf der Trägerlage des mechanischen Verschlusselements mehrere männliche Befestigungselemente angeordnet, üblicherweise in einer Dichte von 10/cm² bis 5000/cm², bevorzugt 200/cm² bis 1000/cm². Eine solche Mehrzahl von männlichen Befestigungselementen bildet dabei die (vorderseitige) Eingriffsfläche des mechanischen Verschlusselements.

Die Trägerlage, deren Rückseite die (rückseitige) Befestigungsfläche bildet, kann im Wesentlichen aus jedem zur Filmherstellung geeigneten thermoplastischen Material ausgebildet sein. Bevorzugte thermoplastische Materialien umfassen Polyester wie beispielsweise Poly(ethylenterephthalat), Polyamide wie beispielsweise Nylon, Poly(styrolacrylonitril), Poly(acrylonitril-butadienstyrol), Polyolefine wie beispielsweise Polypropylen und weichmacherhaltiges Polyvinylchlorid. Die Trägerlage kann aus einem einzigen Material bestehen und einen im Wesentlichen gleichförmigen Aufbau in CD aufweisen, sie kann jedoch auch eine Abfolge von zwei oder mehreren Zonen in CD mit verschiedenen Eigenschaften aufweisen, wobei sich solche Zonen vorzugsweise kontinuierlich in MD erstrecken. Die Trägerlage kann beispielsweise aus Schichten von verschiedenen Materialien ausgebildet sein, die coextrudiert oder aneinander laminiert sind. Die Trägerlage eines mechanischen Verschlusselements umfasst bevorzugt dasselbe Material wie die darauf aufgebrachten männlichen Befestigungselemente und ist mit Letzteren bevorzugt integral ausgebildet. Besonders vorteilhaft sind die Trägerlage und die darauf befindlichen männlichen Befestigungselemente integral aus Polyethylen (PE) oder Polypropylen (PP) ausgebildet.

Die Trägerlage ist üblicherweise flach und hat bevorzugt eine durchschnittliche Dicke zwischen 10 µm und 1 mm, besonders bevorzugt zwischen 15 µm und 750 µm, insbesondere zwischen 20 µm und 500 µm. Die Dicke wird bevorzugt derart gewählt, dass die Trägerlage dick genug ist, um den darauf angebrachten männlichen Befestigungselementen einen ausreichenden Halt zu bieten und insbesondere ein Ausreißen zu vermeiden, und andererseits dünn genug, um nicht zu starr auszufallen, was die Verarbeitbarkeit des Windelverschlussbands und den späteren Tragekomfort der Windel auf Grund der mangelnden Verformbarkeit der Trägerlage beeinträchtigen könnte.

Zwischen dem Substrat und dem mechanischen Verschlusselement, genauer der rückseitigen Befestigungsfläche des mechanischen Verschlusselements, befindet sich ein Klebstoffelement. Das Klebstoffelement umfasst Klebstoff, der vorzugsweise ausgewählt ist aus Heißsiegel-Klebstoffen, insbesondere polyolefinischen Heißsiegel-Klebstoffen, HMPSA (Hot Melt Pressure Sensitive Adhesives) auf Basis natürlichen und synthetischen Kautschuks und/oder Polyacrylat Hotmelts (Heißklebestoffen). Geeignete Basispolymere für den Klebstoff umfassen Polyamide (PA), Polyethylen (PE), amorphe Polyalphaolefine (APAO), Ethylenvinylacetat-Copolymere (EVAC), Polyester-Elastomere (TPE-E), Polyuretan-Elastomere (TPE-U), Copolyamid-Elastomere (TPE-A), Styrol-Block-Copolymer-Elastomer (TPE-S) sowie Harze wie beispielsweise Terpene, Kohlenwasserstoffharze und Kolophonium. Bei den Klebstoffen kann es sich um chemisch härtende Klebstoffe, reaktive Schmelzklebstoffe, physikalisch abbindende Klebstoffe und/oder vernetzbare Klebstoffe, beispielsweise UV-vernetzbare Klebstoffe oder thermisch vernetzbare Klebstoffe, handeln. Der Klebstoff ist vorzugsweise lösungsmittelfrei und hat vorzugsweise die Eigenschaft, in erwärmten Zustand eine geringere Viskosität aufzuweisen als in abgekühltem Zustand. Durch die geringere Viskosität in erwärmten Zustand kann eine ausreichende Benetzung der zu klebenden Flächen gewährleistet werden, welche für eine Anhaftung des Klebstoffs grundlegend sein kann. Durch das Abkühlen und/oder Aushärten kann die gewünschte Klebewirkung beispielsweise durch Adhäsion oder Kohäsion erreicht werden. Der Klebstoff kann demgemäß geeignet sein, in erwärmten Zustand auf die zu klebenden Flächen aufgetragen zu werden, um beim Abkühlen auf Raumtemperatur die gewünschte Klebeverbindung herzustellen. Die zu klebenden Flächen können hierzu vorab einer Vorbehandlung unterzogen werden, wie beispielsweise einer Koronavorbehandlung oder einer Flammvorbehandlung. Der Klebstoff wird üblicherweise im Bereich zwischen 120 und 220 °C, bevorzugt zwischen 140 und 180 °C verarbeitet. Er wird bevorzugt derart ausgewählt, dass er bei Raumtemperatur möglichst vollständig ausgehärtet ist und die freiliegenden Klebstoffflächen keine oder nur noch eine geringe Restklebrigkeit aufweisen.

Das Klebstoffelement wird bevorzugt bereitgestellt, indem Klebstoff auf die rückseitige Befestigungsfläche des mechanischen Verschlusselements aufgebracht wird. Dadurch wird verhindert, dass der Klebstoff in das Substratmaterial absacken kann, bevor eine ausreichende Klebeverbindung zu den jeweiligen mechanischen Verschlusselementen hergestellt wurde. Ein solches Absacken des Klebstoffs wäre insbesondere dann problematisch, wenn das Substrat aus einem saugfähigen Material wie beispielsweise einem Vlies, insbesondere einem unbefilmten Vlies gebildet ist. Umgekehrt nimmt ein solches Substratmaterial den Klebstoff, der bereits auf das mechanische Verschlusselement aufgetragen wurde, besonders gut auf, was zu einer besonders vorteilhaften Verbindung zwischen mechanischem Verschlusselement und Substrat beiträgt. Besonders bei einer Konstellation von saugfähigem Substratmaterial und nicht saugfähigem Verschlusselementmaterial führt das Verfahren daher zu einer erheblichen Verbesserung der Klebewirkung bzw. einer erheblichen Verringerung der benötigten Klebstoffmenge gegenüber herkömmlichen Verfahren. Eine Verringerung der Klebstoffmenge wiederum hat zur Folge, dass im ausgehärteten Zustand weniger hartes Material im Windelverschlussband vorhanden ist, was der Verformbarkeit und damit der Anpassungsfähigkeit des Windelverschlussbands an statische oder dynamische Verformungen zuträglich ist und folglich dessen Haltekraft gegenüber der Landing-Zone verbessert. Mögliche saugfähige Substratmaterialien sind dabei solche Materialien, die den flüssigen erwärmten Klebstoff von der Materialoberfläche weg in das Materialinnere aufsaugen, wie beispielsweise Vliese, insbesondere unbefilmte Vliese, und Papier. Nicht saugfähige Materialien sind solche Materialien, die den flüssigen erwärmten Klebstoff auf ihrer Oberfläche halten, wie beispielsweise Polyethylen (PE) und Polypropylen (PP).

Erfindungsgemäß wird das Klebstoffelement derart bereitgestellt, dass es die rückseitige Befestigungsfläche des mechanischen Verschlusselements nicht vollständig bedeckt. Demgemäß ist zumindest eine äußere Begrenzung des mechanischen Verschlusselements zumindest abschnittsweise klebstofffrei. Vorzugsweise erstreckt sich das Klebstoffelement nicht in CD bis zu einer äußeren Begrenzung des mechanischen Verschlusselements. Insbesondere ist daher eine äußere Begrenzung in CD, bevorzugt beide äußeren Begrenzungen in CD des mechanischen Verschlusselements klebstofffrei. Dies erleichtert eine Führung bzw. Positionierung des mechanischen Verschlusselements, insbesondere in einem kontinuierlichen Verfahren. Es wird nämlich sichergestellt, dass eine Führungs- bzw. Positionierhilfe das mechanische Verschlusselement kontaktieren und damit führen bzw. positionieren kann, ohne zugleich mit Klebstoff in Kontakt zu kommen. Dadurch wird eine Verunreinigung der Führungs- bzw. Positionierhilfe durch Klebstoff vermieden. Aufwändiges Entfernen des Klebstoffs, insbesondere wenn dieser bereits ausgehärtet ist, von solchen Führungs- bzw. Positionierhilfen kann damit entfallen.

Des Weiteren eröffnet sich dadurch die Möglichkeit, dass das Klebstoffelement nach dem Verbinden des klebstoffbeschichteten mechanischen Verschlusselements mit dem Substrat eine räumliche Ausdehnung in der Ebene des Substrats aufweist, die geringer ist als die räumliche Ausdehnung des mechanischen Verschlusselements in der Ebene des Substrats. Unter der räumlichen Ausdehnung in der Ebene des Substrats ist dabei die Fläche zu verstehen, die das jeweilige Element in der Ebene des Substrats, betrachtet aus Richtung der Z-Achse, abdeckt.

Diese Ausgestaltung hat erhebliche Vorteile in Bezug auf die Verformbarkeit des Windelverschlussbands und damit auf die resultierende Haltekraft gegenüber der Landing-Zone. Wie bereits erläutert, kann der Klebstoff nach dem Aushärten einen Bereich bilden, der im Wesentlichen starr ist und sich nicht oder nur mäßig verformen lässt. Die Bereiche des Windelverschlussbands, die mit Klebstoff benetzt sind, stehen dann einer Verformbarkeit des Windelverschlussbands aus der Substratebene heraus entgegen. Sie verhindern oder verringern damit die bereits erläuterten, mit der Verformbarkeit zusammenhängenden Vorteile wie die Anpassungsfähigkeit des Windelverschlussbands an statische oder dynamische Verformungen der Landing-Zone und die daraus resultierende verbesserte Haltewirkung. Damit ist es an sich geboten, die Klebstoffbereiche so klein wie möglich zu halten, jedoch groß genug, dass eine ausreichend feste Verbindung zwischen Substrat und mechanischem Verschlusselement gewährleistet ist, um ein unerwünschtes Ablösen des mechanischen Verschlusselements vom Substrat zu verhindern.

Hinzu kommt, dass üblicherweise auch das mechanische Verschlusselement nur über eine geringe Verformbarkeit verfügt. Eine Begrenzung der Klebstoffelemente auf Bereiche, die vom Verschlusselement vollständig verdeckt werden, stellt zum einen sicher, dass dem Windelverschlussband keine zusätzlichen starren Bereiche verliehen werden. Andererseits trägt diese Ausgestaltung sogar dazu bei, dass die versteifende Wirkung der mechanischen Verschlusselemente auf das Windelverschlussband reduziert wird. Das Substrat wird nämlich nicht über die gesamte räumliche Ausdehnung des mechanischen Verschlusselements, insbesondere in CD, versteift, sondern lediglich über die räumliche Ausdehnung des Klebstoffelements, insbesondere in CD. Dadurch, dass das Klebstoffelement eine räumliche Beabstandung zwischen Substrat und mechanischem Verschlusselement in Richtung der Z-Achse bewirken kann, und selbst nur einen geringeren Anteil des Substrats, in insbesondere in CD, versteift, bleiben größere Bereiche des Substrats frei von versteifenden Einflüssen, was die Verformbarkeit des Windelverschlussbands in dem Bereich, der die mechanischen Verschlusselemente aufweist und der damit für die Entfaltung der Haltewirkung gegenüber der Landing-Zone von entscheidender Bedeutung ist, erheblich verbessert.

Die Größe des Klebstoffelements wird dabei so gewählt, dass eine ausreichende Verbindung zwischen Substrat und mechanischem Verschlusselement gewährleistet ist. Bevorzugt beträgt die räumliche Ausdehnung des Klebstoffelements in der Ebene des Substrats zumindest 50 %, insbesondere zwischen 60 % und 90 %, bevorzugt zwischen 70 % und 80 % der räumlichen Ausdehnung des mechanischen Verschlusselements in der Ebene des Substrats. Nach dem Aufbringen des Klebstoffelements auf die rückseitige Befestigungsfläche des mechanischen Verschlusselements und/oder nach dem Verbinden des klebstoffbeschichteten mechanischen Verschlusselements mit dem Substrat sind also vorzugsweise zumindest 50 %, insbesondere zwischen 60 % und 90 %, bevorzugt zwischen 70 % und 80 % der rückseitigen Befestigungsfläche des mechanischen Verschlusselements durch Klebstoff bedeckt. Weiterhin bevorzugt weist das Klebstoffelement eine Dicke, d.h., eine räumliche Ausdehnung in Richtung der Z-Achse auf, die eine gewisse Beabstandung zwischen Substrat und mechanischem Verschlusselement in Richtung der Z-Achse bewirken kann. Beispielsweise kann das Klebstoffelement eine Dicke zwischen 1 µm und 1 mm, bevorzugt zwischen 10 µm und 500 µm, insbesondere zwischen 20 µm und 100 µm aufweisen.

Bevorzugt wird das Verfahren zur Herstellung des Windelverschlussbands kontinuierlich durchgeführt. Das Substrat kann dazu als Endlosmaterial in Maschinenrichtung über eine Kontaktvorrichtung, beispielsweise ein Paar von horizontal parallel übereinander angeordneten Rollen geführt werden. Die mechanischen Verschlusselemente können ebenfalls als Endlosmaterial oberhalb der Substratbahn entgegen der Maschinenrichtung zugeführt werden und mittels der oberen Kontaktrolle umgelenkt und in Maschinenrichtung mit dem Substrat in Kontakt gebracht werden. Vor dem Umlenken können die mechanischen Verschlusselemente mit Klebstoff beschichtet werden, der ebenfalls kontinuierlich durch eine Düsenanordnung bereitgestellt wird. Die mechanischen Verschlusselemente werden als Streifen bereitgestellt, die sich in MD theoretisch endlos erstrecken. Auf jeden der Streifen wird in MD kontinuierlich Klebstoff aufgebracht, was in einem ebenfalls streifenförmigen Klebstoffelement resultiert. Der Klebstoffauftrag erfolgt derart, dass die Breite des resultierenden Klebstoffelements in CD geringer ist als die Breite des entsprechenden mechanischen Verschlusselements in CD. Das mechanische Verschlusselement überragt das Klebstoffelement in CD auf zumindest einer Seite, besonders bevorzugt auf beiden Seiten. In einer Ausführungsform beträgt die Breite des Klebstoffelements in CD zumindest 50 %, insbesondere zwischen 60 % und 90 %, bzw. zwischen 70 % und 80 % der Breite des entsprechenden mechanischen Verschlusselements in CD. Bevorzugt ist das Klebstoffelement in CD mittig auf dem mechanischen Verschlusselement angeordnet.

Optional kann das kontinuierliche Verfahren weiterhin das Aufbringen von einer oder mehreren (funktionellen) Lagen umfassen, beispielsweise durch Laminieren, Kaschieren oder lösbares Verbinden.

Das aus dem kontinuierlichen Verfahren resultierende Produkt ist ein Windelverschlussband mit theoretisch unendlicher Erstreckung in MD, das auf Scheiben oder Spulen gewickelt werden kann. Ein Konfektionieren, d.h., Schneiden eines solchen Endlos-Windelverschlussbands in CD, bevorzugt in Abständen entlang MD zwischen 2,5 und 5,0 cm, insbesondere 3,0 bis 3,5 cm, ergibt Windelverschlussbänder, die sich insbesondere für ein Verbauen bei Baby- und Inkontinenzwindeln eignen. Das Schneiden kann dabei auf jede geeignete und dem Fachmann bekannte Weise erfolgen, insbesondere mit Hilfe von Rotationsmessern.

Das Bereitstellen der Mehrzahl von mechanischen Verschlusselementen erfolgt dadurch, dass ein einzelnes mechanisches Verschlusselement in mehrere mechanische Verschlusselemente geteilt wird. Dies kann auf jede geeignete und dem Fachmann bekannte Weise erfolgen, beispielsweise durch Schneiden oder Stanzen des einzelnen mechanischen Verschlusselements, insbesondere mit Hilfe von Klingen, Rotationsmessern oder mittels berührungslosen Schneidens wie beispielsweise Laserschneiden und Wasserstrahlschneiden. Ein (berührungsloses) Schneiden bietet sich dabei insbesondere bei einem kontinuierlichen Verfahren an, wobei aus dem einzelnen mechanischen Verschlusselement mehrere Streifen von mechanischen Verschlusselementen resultieren. Ein Stanzen des mechanischen Verschlusselements wiederum kann zu beliebigen Formen führen, wie beispielsweise Dreiecken, Kreisen oder Rauten. Bevorzugt erfolgt das Teilen des mechanischen Verschlusselements vor dessen Beschichtung mit Klebstoff. Dadurch wird auf einfache und vorteilhafte Weise sichergestellt, dass ein verwendetes Schneid- oder Stanzgerät nicht mit Klebstoff in Kontakt kommt. Somit kann eine Verunreinigung des Schneid- oder Stanzgeräts und eine damit verbundene notwendige Reinigung von flüssigem oder bereits ausgehärtetem Klebstoff vermieden werden. Dies verkürzt erheblich die Wartungszeiten und trägt zu einer Verfahrensökonomie bei. Jedoch kann das Teilen des mechanischen Verschlusselements auch nach dessen Beschichtung mit Klebstoff erfolgen. Eine Verunreinigung von verwendetem Schneid- oder Stanzgerät kann in diesem Fall dadurch vermieden werden, dass der Klebstoff nicht in den Bereichen aufgetragen wird, in denen die Teilung des mechanischen Verschlusselements erfolgen soll. Das heißt, das klebstoffbeschichtete mechanische Verschlusselement wird entlang klebstofffreier Bereiche geteilt, insbesondere geschnitten oder gestanzt. Daraus resultiert eine Mehrzahl von mechanischen Verschlusselementen mit jeweils einem Klebstoffelement auf der rückseitigen Befestigungsfläche, das die rückseitige Befestigungsfläche nicht vollständig bedeckt. In einem kontinuierlichen Verfahren können beispielsweise auf einen breiten, als Endlosmaterial bereitgestellten Streifen des mechanischen Verschlusselements mehrere Spuren Klebstoff aufgetragen werden. Dies ergibt zunächst mehrere streifenförmige Klebstoffelemente, die sich auf dem mechanischen Verschlusselement entlang MD erstrecken. Anschließend kann das mechanische Verschlusselement entlang der Bereiche zwischen den streifenförmigen Klebstoffelementen geteilt werden.

Die Trennlinien zwischen den einzelnen Streifen der mechanischen Verschlusselemente müssen nicht geradlinig verlaufen, d.h., sie können z.B. regelmäßige oder unregelmäßige Abweichungen quer und längs zur Maschinenlaufrichtung aufweisen. Die mechanischen Verschlusselemente müssen nicht durchgängig durchtrennt nach dem Schneidvorgang vorliegen, d.h. sie können noch Stege aus nicht durchtrenntem mechanischem Verschlussmaterial aufweisen.

Der Auftrag des Klebstoffs kann, insbesondere in einem kontinuierlichen Verfahren, mittels einer Beschichtungsvorrichtung erfolgen, die eine Düsenanordnung mit einer Mehrzahl von Düsenöffnungen umfasst. Die Düsenanordnung kann einen Düsenkörper und eine Düsenlippe aufweisen, in denen miteinander kommunizierende Kanäle vorgesehen sind. In den kommunizierenden Kanälen kann in den Düsenkörper eingebrachter Klebstoff zur Düsenlippe transportiert werden und hier die Düsenanordnung über eine Mehrzahl von Düsenöffnungen verlassen. Die Düsenlippe ist demgemäß der Bereich der Düsenanordnung, der während des Klebstoffauftrags auf die mechanischen Verschlusselemente mit den mechanischen Verschlusselementen in enger räumlicher Beziehung steht und diese unter Umständen berührt. Vorteilhaft weist die Düsenlippe daher an einer Oberfläche einen Kontaktbereich auf, der über besondere Eigenschaften wie Widerstandsfähigkeit gegen mechanische Einwirkung, Gleiteigenschaften, schmutzabweisende Eigenschaften, antistatische Eigenschaften etc. verfügt. Besonders bevorzugt ist im Kontaktbereich ein auswechselbares Kontaktelement mit eben solchen Eigenschaften vorgesehen. Bevorzugt kann die Düsenlippe jeweils im Bereich einer Düsenöffnung eine Aussparung aufweisen, die sich entlang MD erstreckt, vorzugsweise über den gesamten Bereich der Düsenlippe entlang MD. Die Breite der Aussparung in CD kann dem Durchmesser der Düsenöffnung in CD entsprechen.

Die Beschichtungsvorrichtung umfasst ferner eine Führungshilfe zum Führen eines oder mehrerer mechanischer Verschlusselemente. Dadurch kann einerseits sichergestellt werden, dass der Klebstoff bezüglich des (jeweiligen) mechanischen Verschlusselements korrekt positioniert wird und andererseits gemäß der gewünschten räumlichen Ausdehnung des Klebstoffelements korrekt dosiert wird. Dazu kann die Führungshilfe eine oder mehrere Aussparungen zum Führen einer korrespondierenden Anzahl von mechanischen Verschlusselementen umfassen. Der Durchmesser der (jeweiligen) Aussparung in CD wird dabei derart gewählt, dass er mit der Breite des darin zu führenden (jeweiligen) mechanischen Verschlusselements korrespondiert. Die Aussparungen können ferner derart dimensioniert sein, dass sie in ihrer Tiefe vorzugsweise zumindest 70 %, insbesondere zwischen 70% und 250%, bevorzugt zwischen 80 % und 110 %, der Höhe des darin zu führenden mechanischen Verschlusselements entsprechen. Die Führungshilfe kann eine Mehrzahl von Aussparungen zum Führen einer korrespondierenden Mehrzahl von mechanischen Verschlusselementen umfassen, und die Düsenanordnung kann eine korrespondierende Mehrzahl von Düsenöffnungen zum Aufbringen der Klebstoffelemente umfassen. Die Düsenöffnungen sind dabei korrespondierend zur Position der Aussparungen in der Führungshilfe angeordnet. Die Aussparungen in der Führungshilfe stellen vorteilhaft sicher, dass sich jedes der mechanischen Verschlusselemente zum Zeitpunkt des Klebstoffauftrags in einer korrekten Position bezüglich der jeweiligen Düse befindet. Die Aussparung hat dabei den Vorteil, dass sie eine mögliche Bewegung eines mechanischen Verschlusselements in drei Richtungen verhindern kann. Diese Richtungen sind bei einer bevorzugten Anordnung der Führungshilfe ein Versatz des mechanischen Verschlusselements in CD nach links, in CD nach rechts und in Richtung der Z-Achse von der Düsenöffnung weg oder zur Düsenöffnung hin. Damit wird ein korrekt positionierter und gleichmäßig dosierter Klebstoffauftrag ermöglicht. Der Durchmesser jeder Düsenöffnung in CD ist so gewählt werden, dass er geringer ist als der Durchmesser der jeweiligen korrespondierenden Aussparung in der Führungshilfe in CD. Bevorzugt wird der Durchmesser der Düsenöffnung in CD so gewählt, dass er zumindest 50 %, insbesondere zwischen 60 % und 90 %, bevorzugt zwischen 70 % und 80 % des Durchmessers der jeweiligen korrespondierenden Aussparung der Führungshilfe in CD beträgt. Damit wird auf vorteilhafte Weise ermöglicht, dass der aufgebrachte Klebstoff insbesondere in einem kontinuierlichen Verfahren eine entsprechende Fläche der rückseitigen Befestigungsfläche des mechanischen Verschlusselements bedeckt.

Die Führungshilfe und die Düsenanordnung können als zwei separate Bauelemente ausgebildet sein. In diesem Fall ist zumindest eines der beiden Bauelemente derart konfiguriert, dass es in die räumliche Nähe zu dem jeweils anderen Bauelement gebracht werden kann. Beispielsweise kann die Führungshilfe aus einer Platte oder einem Block ausgebildet sein, worin Aussparungen vorhanden sind. Die Aussparungen können derart dimensioniert sein, dass sie in ihrem Durchmesser in CD der Breite des jeweiligen mechanischen Verschlusselements in CD entsprechen und in ihrer Tiefe vorzugsweise zumindest 70 %, insbesondere zwischen 70% und 250%, bevorzugt zwischen 80 % und 110 %, der Höhe des mechanischen Verschlusselements entsprechen. Die Düsenanordnung wiederum kann Düsenöffnungen und optional zugeordnete Aussparungen in der Düsenlippe und dem optionalen Kontaktelement aufweisen, deren jeweiliger Durchmesser in CD geringer ist als der Durchmesser der Aussparungen in der Führungshilfe in CD, vorzugsweise zumindest 50 %, insbesondere zwischen 60 % und 90 %, bevorzugt zwischen 70 % und 80 % des Durchmessers der jeweils korrespondierenden Aussparung in der Führungshilfe.

Bevorzugt sind die Düsenanordnung und die Führungshilfe integral ausgebildet. Insoweit dient die Düsenanordnung zugleich dazu, die mechanischen Verschlusselemente zu führen und für den Klebstoffauftrag korrekt zu positionieren. Dies kann dadurch erfolgen, dass in der Düsenlippe und/oder dem optionalen Kontaktelement zusätzlich zu den Düsenöffnungen und den optional zugeordneten Aussparungen auch Aussparungen zum Führen der mechanischen Verschlusselemente vorhanden sind. Da jeder Aussparung, die als Führungshilfe dient, eine Düsenöffnung mit optional zugeordneter Aussparung zugeordnet ist, und die Aussparung für die Führungshilfe vorzugsweise in CD breiter ist als die korrespondierende Düsenöffnung bzw. zugeordnete Aussparung, resultiert dies in einer T-Form des Querschnitts durch Düsenöffnung und Führungshilfe. Die Tiefe der Aussparung, die als Führungshilfe dient, d.h. der Querbalken des T, kann dabei so gewählt sein, dass sie zumindest 70 %, vorzugsweise zwischen 70% und 250%, bevorzugt zwischen 80 % und 110 % der Höhe des jeweils verwendeten mechanischen Verschlusselements entspricht. Die Düsenöffnungen bzw. zugeordneten Aussparungen wiederum können einen Durchmesser in CD aufweisen, der geringer ist als der Durchmesser der Aussparungen, die als Führungshilfe dienen, vorzugsweise zumindest 50 %, insbesondere zwischen 60 % und 90 %, bevorzugt zwischen 70 % und 80 % des Durchmessers der jeweils korrespondierenden Aussparung, die als Führungshilfe dient. Das mechanische Verschlusselement kann dabei auf verschiedene Weise innerhalb der Aussparung gehalten werden, beispielsweise durch Halten des mechanischen Verschlusselements unter Spannung gegen die Führungshilfe oder durch Eindrücken des mechanischen Verschlusselements in die Führungshilfe mittels eines gegengeordneten Bauelements wie beispielsweise einer Rolle.

Bevorzugt umfasst die Vorrichtung zur Herstellung eines Windelverschlussbands ferner eine Schneidvorrichtung zum Schneiden der mechanischen Verschlusselemente, wie beispielsweise eine Anordnung von Klingen oder berührungslosen Schneidwerkzeugen wie beispielsweise Laser-Schneidwerkzeugen und Wasserstrahl-Schneidwerkzeugen. Damit ist es insbesondere in einem kontinuierlichen Verfahren möglich, ein mechanisches Verschlusselement - sei es nach dessen Beschichtung mit Klebstoff oder vor dessen Beschichtung mit Klebstoff - in eine Mehrzahl von mechanischen Verschlusselementen wie oben beschrieben zu teilen. Je nach durchzuführendem Verfahren ist daher die optionale Schneidvorrichtung vorteilhaft entweder der Beschichtungsvorrichtung nachgeordnet (Schneiden der klebstoffbeschichteten mechanischen Verschlusselemente) oder der Beschichtungsvorrichtung vorgelagert (Schneiden der unbeschichteten mechanischen Verschlusselemente).

Bevorzugt zeichnet sich die Vorrichtung zur Herstellung eines Windelverschlussbands weiterhin dadurch aus, dass sie es ermöglicht, dass das Klebstoffelement nach dem Verbinden des mechanischen Verschlusselements mit dem Substrat eine räumliche Ausdehnung in der Ebene des Substrats aufweist, die geringer ist als die räumliche Ausdehnung des mechanischen Verschlusselements in der Ebene des Substrats. Dies kann insbesondere dadurch bewirkt werden, dass die Kontaktvorrichtung, die das rückseitig mit Klebstoff beschichtete mechanische Verschlusselement mit dem Substrat in Verbindung bringt, einen variablen Anpressdruck ausüben kann. Damit ist es möglich, dass das klebstoffbeschichtete mechanische Verschlusselement mit variierender Intensität gegen das Substrat gepresst wird, was einerseits eine Beeinflussung der Anhaftung des Klebstoffs am Substrat und andererseits eine Beeinflussung der Verteilung des Klebstoffs in der Ebene des Substrats erlaubt. Bevorzugt wird der Anpressdruck so gewählt, dass sowohl eine ausreichende Haftverbindung zwischen Klebstoff und Substrat, als auch eine räumliche Ausdehnung des Klebstoffelements in der Ebene des Substrats gewährleistet ist, die geringer ist als die räumliche Ausdehnung des mechanischen Verschlusselements in der Ebene des Substrats. Die Kontaktvorrichtung ist demgemäß vorzugsweise aus einem Paar von horizontal parallel übereinander angeordneten Rollen ausgebildet, deren gegenseitiger Anpressdruck variiert werden kann.

Die Erfindung wird im Folgenden durch Bezugnahme auf die angehängten Figuren beispielhalber beschrieben, in denen gilt:
Figur 1 ist eine Draufsicht einer exemplarischen Ausführungsform eines Windelverschlussbands gemäß der Erfindung;
Figur 2 ist eine Schnittansicht entlang der Linie A-A in Fig. 1;
Figur 3 ist eine perspektivische Darstellung einer exemplarischen Düsenanordnung zur Bereitstellung einer Mehrzahl von Klebstoffelementen;
Figur 4 ist eine schematische Seitenansicht der exemplarischen Düsenanordnung der Figur 3;
Figur 5 ist eine Schnittansicht entlang der Linie A-A in Fig. 4;
Figur 6 ist eine schematische Seitenansicht einer Beschichtungsvorrichtung mit einer Führungshilfe und einer separaten Düsenanordnung gemäß einer Ausführungsform der Erfindung;
Figur 7 ist eine schematische Seitenansicht einer Beschichtungsvorrichtung gemäß einer weiteren Ausführungsform der Erfindung, bei der die Führungshilfe und die Düsenanordnung integral ausgebildet sind;
Figur 8 ist eine schematische Darstellung einer exemplarischen Vorrichtung zur Herstellung eines Windelverschlussbands gemäß einer Ausführungsform der Erfindung; und
Figur 9 ist eine schematische Ansicht einer Windel, an deren Windelohren jeweils ein Windelverschlussband gemäß einer bevorzugten Ausführungsform der Erfindung angebracht ist.

Figur 1 zeigt eine exemplarische Ausführungsform eines Windelverschlussbands gemäß der Erfindung. Genauer zeigt Figur 1 einen Vorläufer, aus dem durch Schneiden entlang der Linie S zwei Windelverschlussbänder 1 erhalten werden, wie sie sich für ein Verbauen an einer Windel eignen. Jedes dieser beiden so erhaltenen Windelverschlussbänder weist ein Substrat 2 auf, das sich über einen Nutzerbereich 4 und einen Haltebereich 5 erstreckt. Das Substrat 2 ist aus einer Vlies-Lage gebildet, die sich in einer Ebene erstreckt, die durch MD und CD definiert wird. Im Nutzerbereich 4 sind auf dem Substrat 2 mechanische Verschlusselemente 3 angeordnet. Jedes der mechanischen Verschlusselemente 3 verdeckt ein darunter befindliches Klebstoffelement (nicht gezeigt), das sich zwischen dem mechanischen Verschlusselement 3 und dem Substrat 2 befindet.

Figur 2 ist eine Schnittansicht entlang der Linie A-A in Figur 1. Darin zu erkennen sind neben dem Substrat 2 und den mechanischen Verschlusselementen 3 auch dazwischen angeordnete Klebstoffelemente 6. Die Klebstoffelemente 6 weisen entlang CD eine geringere räumliche Ausdehnung auf als die mechanischen Verschlusselemente.

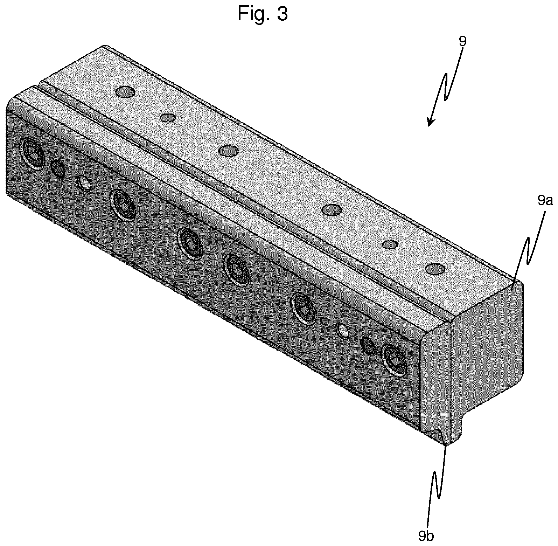

Figur 3 zeigt eine perspektivische Darstellung einer exemplarischen Düsenanordnung 9 zur Bereitstellung einer Mehrzahl von Klebstoffelementen. Die Düsenanordnung 9 umfasst einen Düsenkörper 9a und eine Düsenlippe 9b. Die Düsenanordnung 9 in der dargestellten Form ist zweiteilig ausgebildet und kann entlang einer sowohl durch den Düsenkörper 9a als auch die Düsenlippe 9b verlaufenden Trennlinie zerlegt werden. Im Inneren des Düsenkörpers 9a und der Düsenlippe 9b erstrecken sich miteinander kommunizierende Kanäle (nicht gezeigt). In den kommunizierenden Kanälen kann in den Düsenkörper eingebrachter Klebstoff zur Düsenlippe transportiert werden und hier die Düsenanordnung über eine Mehrzahl von Düsenöffnungen verlassen.

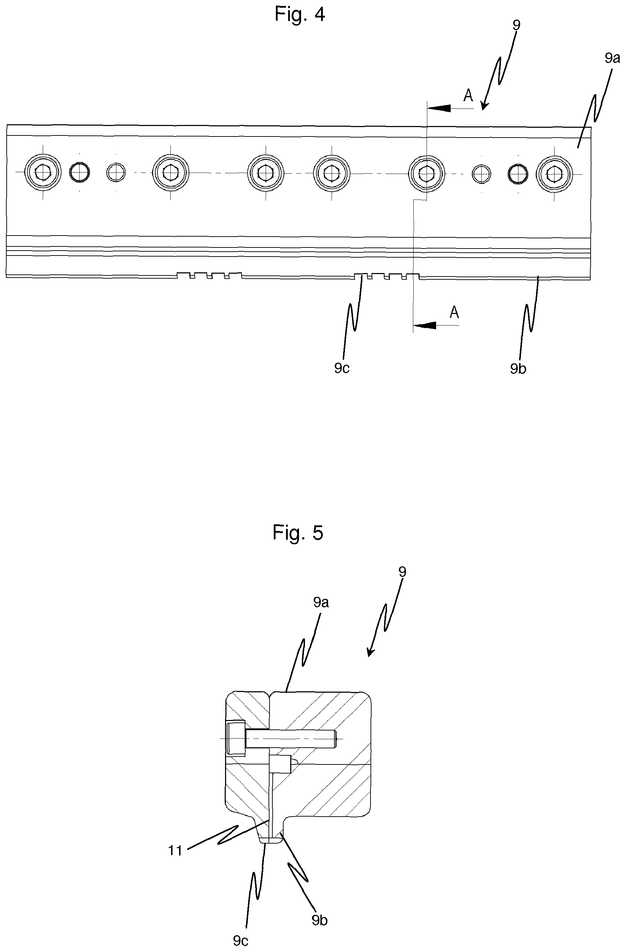

Figur 4 zeigt eine schematische Seitenansicht der exemplarischen Düsenanordnung 9 der Figur 3, umfassend den Düsenkörper 9a und die Düsenlippe 9b. Im unteren Bereich der Düsenlippe 9b befinden sich Aussparungen 9c, die jeweils einer Düsenöffnung (nicht gezeigt) zugewiesen sind und sich über die Gesamte Tiefe der Düsenlippe in MD erstrecken.

Figur 5 zeigt eine Schnittansicht entlang der Linie A-A in Fig. 4. Gezeigt sind die Düsenanordnung 9, umfassend den Düsenkörper 9a und die Düsenlippe 9b, sowie eine Düse 11, die aus einem Kanal gebildet ist, der sich durch den Düsenkörper 9a und die Düsenlippe 9b erstreckt. Im unteren Bereich der Düsenlippe 9b ist eine Aussparung 9c gezeigt, die der Düse 11 zugewiesen ist und sich über die Gesamte Tiefe der Düsenlippe in MD erstreckt.

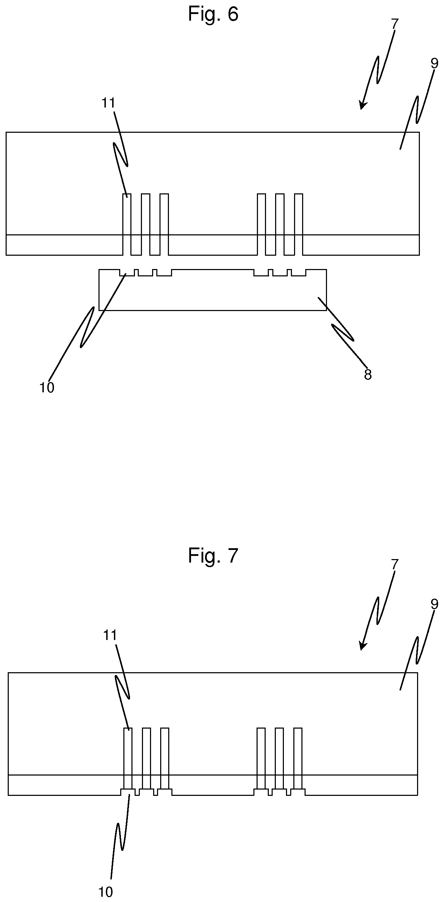

Figur 6 zeigt eine exemplarische Ausführungsform einer Beschichtungsvorrichtung 7. Diese umfasst in der gezeigten Variante eine Führungshilfe 8 zum Führen von mechanischen Verschlusselementen sowie eine Düsenanordnung 9. Die Führungshilfe 8 und die Düsenanordnung 9 weisen jeweils Aussparungen 10 und Düsen 11 auf, die miteinander korrespondieren, d. h. jeder Aussparung 10 in der Führungshilfe 8 ist eine Düse 11 in der Düsenanordnung 9 zugeordnet. Die Aussparung 10 der Führungshilfe 8 weist dabei jeweils einen größeren Durchmesser auf als die korrespondierende Düse 11 in der Düsenanordnung 9. Der Abstand zwischen der Führungshilfe 8 und der Düsenanordnung 9 kann variiert werden. Insbesondere kann die Führungshilfe 8 an die Düsenanordnung 9 herangefahren werden, so dass sie diese kontaktiert oder beinahe kontaktiert.

Figur 7 ist eine schematische Seitenansicht einer Beschichtungsvorrichtung 7 gemäß einer weiteren Ausführungsform der Erfindung. Bei dieser Ausführungsform sind die Führungshilfe 8 und die Düsenanordnung 9 integral ausgebildet. Genauer gesagt weist die Beschichtungsvorrichtung 7 Aussparungen 10 und Düsen 11 auf, die im Querschnitt die Form eines T aufweisen, wobei der jeweils breitere Bereich, d.h., der Querbalken des T, als Führungshilfe dient.

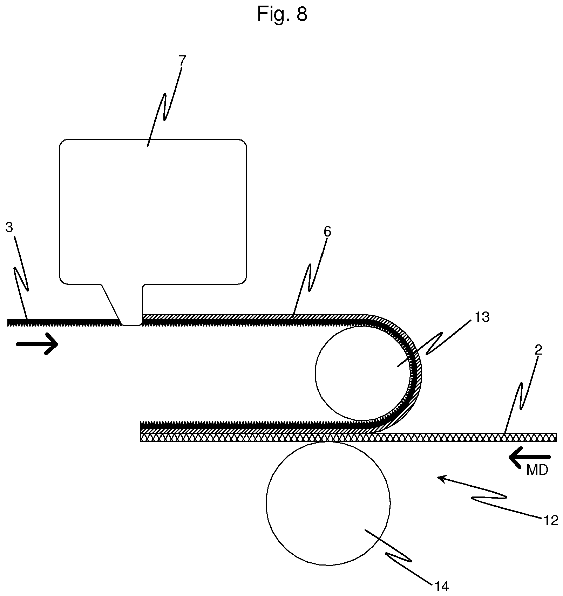

Figur 8 ist eine schematische Darstellung einer exemplarischen Vorrichtung zur Herstellung eines Windelverschlussbands gemäß einer Ausführungsform der Erfindung. Die Vorrichtung umfasst eine Substratzuführung zur Bereitstellung des Substrats 2, eine Verschlusselementzuführung zur Bereitstellung der mechanischen Verschlusselemente 3, eine Beschichtungsvorrichtung 7 zum Aufbringen des Klebstoffelements 6 auf die rückseitige Befestigungsfläche jedes der mechanischen Verschlusselemente 3 sowie eine Kontaktvorrichtung 12 zum Kontaktieren der klebstoffbeschichteten mechanischen Verschlusselemente 3 mit dem Substrat 2. Die Kontaktvorrichtung 12 umfasst eine horizontal angeordnete obere Rolle 13 und eine parallel dazu angeordnete untere Rolle 14. Die untere Rolle 14 transportiert das Substrat 2 in MD. Die mechanischen Verschlusselemente 3 werden oberhalb der Substratbahn entgegen MD zugeführt und mittels der oberen Rolle 13 umgelenkt und in MD mit dem Substrat 2 in Kontakt gebracht. Vor dem Umlenken werden die mechanischen Verschlusselemente 3 mittels der Beschichtungsvorrichtung 7 mit Klebstoff 6 beschichtet.

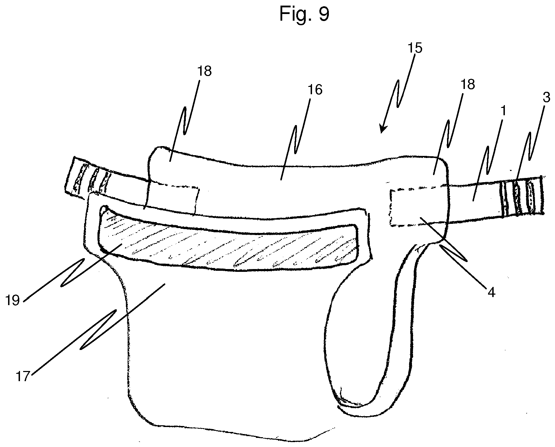

Figur 9 zeigt eine schematische Ansicht einer Windel 15 mit einem hinteren Taillenbereich 16 und einem vorderen Taillenbereich 17. An deren Windelohren 18 ist jeweils ein Windelverschlussband 1 gemäß einer bevorzugten Ausführungsform der Erfindung angebracht. Das Windelverschlussband 1 ist dabei jeweils über seinen Befestigungsbereich 4 mit dem Windelohr 18 verbunden. Der vordere Taillenbereich 17 der Windel 15 weist eine Landing-Zone 19 auf. Diese umfasst weibliche Befestigungselemente, die mit den männlichen Befestigungselementen der mechanischen Verschlusselemente 3 eine lösbare Halteverbindung ausbilden können.

Die Erfindung wurde beispielhalber anhand bevorzugter Ausführungsformen dargestellt. Der Fachmann wird erkennen, dass Abwandlungen hiervon möglich sind, ohne von dem zu Grunde liegenden erfindungsgemäßen Konzept abzuweichen. Die dargestellten Ausführungsformen sollen daher nicht als Einschränkung des Schutzbereichs verstanden werden, der durch die beigefügten Ansprüche definiert wird.

### Bezugszeichenliste

- 1: Windelverschlussband
- 2: Substrat
- 3: Mechanische Verschlusselemente
- 4: Befestigungsbereich
- 5: Nutzerbereich
- 6: Klebstoffelemente
- 7: Beschichtungsvorrichtung
- 8: Führungshilfe
- 9: Düsenanordnung; 9a Düsenkörper; 9b Düsenlippe; 9c Aussparungen
- 10: Aussparung in Führungshilfe
- 11: Düse
- 12: Kontaktvorrichtung
- 13: Obere Rolle
- 14: Untere Rolle
- 15: Windel
- 16: Hinterer Taillenbereich
- 17: Vorderer Taillenbereich
- 18: Windelohr
- 19: Landing-Zone

## Patentansprüche

1. Windelverschlussband, umfassend ein Substrat sowie eine darauf angeordnete Mehrzahl von mechanischen Verschlusselementen mit einer vorderseitigen Eingriffsfläche und einer rückseitigen Befestigungsfläche, **dadurch gekennzeichnet, dass** zwischen dem Substrat und der rückseitigen Befestigungsfläche von jedem mechanischen Verschlusselement jeweils ein Klebstoffelement angeordnet ist, das die rückseitige Befestigungsfläche des mechanischen Verschlusselements nicht vollständig bedeckt, wobei in einer Draufsicht auf das Substrat das Klebstoffelement von dem daran befestigten mechanischen Verschlusselement vollständig verdeckt ist, wobei das mechanische Verschlusselement das Klebstoffelement an mindestens einer Begrenzung überragt.

2. Windelverschlussband nach Anspruch 1, **dadurch gekennzeichnet, dass** das Substrat in einer Zone, in der die Mehrzahl von mechanischen Verschlusselementen angeordnet ist, nicht in der Ebene des Substrats dehnbar ist und/oder eine Zugfestigkeit gemäß DIN EN 29073-3 von mehr als 10 N/50mm, vorzugsweise zwischen 15 und 200 N/50mm, insbesondere zwischen 20 und 150 N/50mm aufweist.

3. Windelverschlussband nach Anspruch 1 oder 2, **dadurch gekennzeichnet, dass** das Substrat in der Zone, in der die Mehrzahl von mechanischen Verschlusselementen angeordnet ist, nicht in CD dehnbar ist.

4. Windelverschlussband nach einem der vorherigen Ansprüche, **dadurch gekennzeichnet, dass** das Klebstoffelement eine räumliche Ausdehnung in der Ebene des Substrats aufweist, die geringer ist als die räumliche Ausdehnung des mechanischen Verschlusselements in der Ebene des Substrats, derart, dass das Substrat in der Zone, in der die Mehrzahl von mechanischen Verschlusselementen angeordnet ist, eine gute Verformbarkeit aus der Substratebene heraus aufweist.

5. Windelverschlussband nach einem der vorherigen Ansprüche, **dadurch gekennzeichnet, dass** das Klebstoffelement zwischen 60 % und 90 %, bevorzugt zwischen 70 % und 80 % der rückseitigen Befestigungsfläche des mechanischen Verschlusselements bedeckt.

6. Windelverschlussband nach einem der vorherigen Ansprüche, **dadurch gekennzeichnet, dass** die mechanischen Verschlusselemente männliche Befestigungselemente umfassen, die sich ausgehend von einer Trägerlage erstrecken.

7. Windelverschlussband nach einem der vorherigen Ansprüche, **dadurch gekennzeichnet, dass** die mechanischen Verschlusselemente aus PE oder PP gebildet sind.

8. Windelverschlussband nach einem der vorherigen Ansprüche, **dadurch gekennzeichnet, dass** das Klebstoffelement einen Klebstoff umfasst, der ausgewählt ist aus polyolefinischen Heißsiegel-Klebstoffen, HMPSA (Hot Melt Pressure Sensitive Adhesives) auf Basis natürlichen und synthetischen Kautschuks und/oder Polyacrylat Hotmelts (Heißklebestoffen).

9. Verfahren zur Herstellung eines Windelverschlussbands gemäß einem der Ansprüche 1 bis 8, umfassend:
(a) Bereitstellen eines Substrats;
(b) Bereitstellen einer Mehrzahl von mechanischen Verschlusselementen mit jeweils einem Klebstoffelement auf einer rückseitigen Befestigungsfläche; und
(c) Verbinden der mechanischen Verschlusselemente mit dem Substrat mittels des Klebstoffelements;
**dadurch gekennzeichnet, dass** das jeweilige Klebstoffelement derart bereitgestellt wird, dass es die rückseitige Befestigungsfläche des mechanischen Verschlusselements nicht vollständig bedeckt, und wobei das Bereitstellen der Mehrzahl von mechanischen Verschlusselementen mit jeweils einem Klebstoffelement auf einer rückseitigen Befestigungsfläche in Schritt b dadurch erfolgt, dass ein mechanisches Verschlusselement in mehrere mechanische Verschlusselemente geteilt wird, und jeweils ein Klebstoffelement auf die rückseitige Befestigungsfläche jedes der resultierenden mechanischen Verschlusselemente aufgebracht wird.

10. Verfahren nach Anspruch 9, **dadurch gekennzeichnet, dass** das Klebstoffelement derart bereitgestellt wird, dass es zwischen 60 % und 90 %, bevorzugt zwischen 70 % und 80 % der rückseitigen Befestigungsfläche des mechanischen Verschlusselements bedeckt.

11. Verfahren nach Anspruch 9 oder 10, **dadurch gekennzeichnet, dass** die Schritte a. bis c. kontinuierlich erfolgen.

12. Vorrichtung zur Herstellung eines Windelverschlussbands gemäß einem der Ansprüche 1 bis 8, umfassend: eine Substratzuführung zur Bereitstellung eines Substrats; eine Verschlusselementzuführung zur Bereitstellung zumindest eines mechanischen Verschlusselements mit einer vorderseitigen Eingriffsfläche und einer rückseitigen Befestigungsfläche; eine Beschichtungsvorrichtung zum Aufbringen von Klebstoff auf die rückseitige Befestigungsfläche des zumindest einen mechanischen Verschlusselements; und eine Kontaktvorrichtung zum Verbinden des zumindest einen mechanischen Verschlusselements mit dem Substrat mittels des Klebstoffs, **dadurch gekennzeichnet, dass** die Beschichtungsvorrichtung eine Düsenanordnung mit einer Mehrzahl von Düsenöffnungen zum Bereitstellen einer korrespondierenden Mehrzahl von Klebstoffelementen umfasst, und wobei dass die Beschichtungsvorrichtung ferner eine Führungshilfe zum Führen des zumindest einen mechanischen Verschlusselements umfasst, und wobei die Führungshilfe eine Mehrzahl von Aussparungen zum Führen einer Mehrzahl von mechanischen Verschlusselementen umfasst, wobei jeder Aussparung in der Führungshilfe eine korrespondierende Düsenöffnung in der Düsenanordnung zugeordnet ist, und wobei jede der Düsenöffnungen einen Durchmesser in CD aufweist, der geringer ist als der Durchmesser der korrespondierenden Aussparung in der Führungshilfe in CD.

13. Vorrichtung nach Anspruch 12, **dadurch gekennzeichnet, dass** die Führungshilfe und die Düsenanordnung integral ausgebildet sind, vorzugsweise in Form einer Baugruppe mit einer Mehrzahl von T-förmigen Aussparungen.

14. Windel, umfassend ein oder mehrere Windelverschlussbänder gemäß einem der Ansprüche 1 bis 8.

## Claims

1. Diaper closing strip comprising a substrate and a plurality of mechanical fasteners arranged thereon having a front engaging surface and a rear attachment surface, **characterised in that** between the substrate and the rear attachment surface of each mechanical fastener a respective adhesive element is arranged that does not entirely cover the rear attachment surface of the mechanical fastener, wherein in a plan view of the substrate the adhesive element is covered fully by the mechanical fastener attached to the latter, wherein the mechanical fastener extends beyond the adhesive element on at least one boundary.

2. Diaper closing strip according to claim 1, **characterised in that** the substrate in an area in which the plurality of mechanical fasteners are arranged cannot stretch in the plane of the substrate and/or has a tensile strength in accordance with DIN EN 29073-3 of more than 10 N/50 mm, preferably between 15 and 200 N/50 mm, in particular between 20 and 150 N/50 mm.

3. Diaper closing strip according to claim 1 or 2, **characterised in that** the substrate in the area in which the plurality of mechanical fasteners are arranged cannot stretch in CD.

4. Diaper closing strip according to any of the preceding claims, **characterised in that** the adhesive element has a spatial extension in the plane of the substrate which is smaller than the spatial extension of the mechanical fastener in the plane of the substrate, such that the substrate in the area in which the plurality of mechanical fasteners are arranged has good deformability from the substrate plane.

5. Diaper closing strip according to any of the preceding claims, **characterised in that** the adhesive element covers between 60 % and 90 %, preferably between 70 % and 80 %, of the rear attachment surface of the mechanical fastener.

6. Diaper closing strip according to any of the preceding claims, **characterised in that** the mechanical fasteners comprise male fastening elements that extend from a supporting layer.

7. Diaper closing strip according to any of the preceding claims, **characterised in that** the mechanical fasteners are made of PE or PP.

8. Diaper closing strip according to any of the preceding claims, **characterised in that** the adhesive element comprises an adhesive, which is selected from polyolefin-based heat-seal adhesives, HMPSA (hot melt pressure sensitive adhesives) based on natural and synthetic rubber and/or polyacrylate hot melt adhesives.

9. Method for producing a diaper closing strip according to any of claims 1 to 8, comprising:
(a) providing a substrate,
(b) providing a plurality of mechanical fasteners each with an adhesive element on a rear attachment surface, and
(c) joining the mechanical fasteners to the substrate by means of the adhesive element,
**characterised in that** the respective adhesive element is provided such that it does not completely cover the rear attachment surface of the mechanical fastener, and wherein the plurality of mechanical fasteners each with an adhesive element on a rear attachment surface in step b is provided by dividing one mechanical fastener into a plurality of mechanical fasteners, and then applying an adhesive element to the rear attachment surface of each of the resultant mechanical fasteners.

10. Method according to claim 9, **characterised in that** the adhesive element is provided such that it covers between 60 % and 90 %, preferably between 70 % and 80 %, of the rear attachment surface of the mechanical fastener.

11. Method according to claim 9 or 10, **characterised in that** steps a to c are performed continuously.

12. Device for producing a diaper closing strip according to any of claims 1 to 8, comprising a substrate feed for providing a substrate, a fastener feed for providing at least one mechanical fastener, having a front engaging surface and a rear attachment surface, a coating device for applying adhesive to the rear attachment surface of the at least one mechanical fastener and a contact device for joining the at least one mechanical fastener to the substrate by means of the adhesive, **characterised in that** the coating device comprises a nozzle arrangement with a plurality of nozzle apertures for providing a corresponding number of adhesive elements, and wherein the coating device also comprises a guiding aid for guiding the at least one mechanical fastener, and wherein the guiding aid comprises a plurality of recesses for guiding a plurality of mechanical fasteners, wherein each recess in the guiding aid is allocated a corresponding nozzle aperture in the nozzle arrangement, and wherein each of the nozzle apertures has a diameter in CD that is less than the diameter of the corresponding recess in the guiding aid in CD.

13. Device according to claim 12, **characterised in that** the guiding aid and the nozzle arrangement are designed to be integral, preferably in the form of an assembly having a plurality of T-shaped recesses.

14. Diaper comprising one or more diaper closing strips according to any of claims 1 to 8.

## Revendications

1. Bande de fermeture de couche-culotte, comprenant un substrat ainsi qu'une multiplicité d'éléments de fermeture mécanique, agencés dessus, avec une surface de mise en prise avant et une surface de fixation arrière, **caractérisée en ce qu'**il est agencé entre le substrat et la surface de fixation arrière de chaque élément de fermeture mécanique à chaque fois un élément adhésif qui ne couvre pas complètement la surface de fixation arrière de l'élément de fermeture mécanique, lequel élément adhésif est complètement couvert, dans une vue de dessus du substrat, par l'élément de fermeture mécanique fixé à lui, lequel élément de fermeture mécanique dépasse de l'élément adhésif au moins au niveau d'une limite.

2. Bande de fermeture de couche-culotte selon la revendication 1, **caractérisée en ce que**, dans une zone dans laquelle les éléments de fermeture mécaniques sont agencés, le substrat n'est pas extensible dans le plan du substrat et/ou présente une résistance à la traction selon la norme DIN EN 29073-3 de plus de 10 N/50 mm, de préférence entre 15 et 200 N/50 mm, en particulier entre 20 et 150 N/50 mm.

3. Bande de fermeture de couche-culotte selon la revendication 1 ou 2, **caractérisée en ce que**, dans la zone dans laquelle les éléments de fermeture mécaniques sont agencés, le substrat n'est pas extensible dans la direction transversale.

4. Bande de fermeture de couche-culotte selon l'une quelconque des revendications précédentes, **caractérisée en ce que** l'élément adhésif présente dans le plan du substrat une extension spatiale qui est plus petite que l'extension spatiale de l'élément de fermeture mécanique dans le plan du substrat de telle sorte que le substrat, dans la zone dans laquelle les éléments de fermeture mécaniques sont agencés, présente une bonne déformabilité hors du plan de substrat.

5. Bande de fermeture de couche-culotte selon l'une quelconque des revendications précédentes, **caractérisée en ce que** l'élément adhésif couvre entre 60% et 90%, de préférence entre 70 % et 80 %, de la surface de fixation arrière de l'élément de fermeture mécanique.

6. Bande de fermeture de couche-culotte selon l'une quelconque des revendications précédentes, **caractérisée en ce que** les éléments de fermeture mécaniques comprennent des éléments de fixation mâles qui s'étendent à partir d'une couche porteuse.

7. Bande de fermeture de couche-culotte selon l'une quelconque des revendications précédentes, **caractérisée en ce que** les éléments de fermeture mécaniques sont formés en PE ou PP.

8. Bande de fermeture de couche-culotte selon l'une quelconque des revendications précédentes, **caractérisée en ce que** l'élément adhésif comprend un adhésif qui est choisi parmi les adhésifs thermofusibles polyoléfiniques, HMPSA (Hot Melt Pressure Sensitive Adhesives) à base de caoutchouc naturel et synthétique et/ou colles thermofusibles à base de polyacrylate (hot melt adhesives).

9. Procédé de fabrication d'une bande de fermeture de couche-culotte selon l'une quelconque des revendications 1 à 8, comprenant :
(a) préparation d'un substrat ;
(b) préparation d'une multiplicité d'éléments de fermeture mécaniques avec à chaque fois un élément adhésif sur une surface de fixation arrière ; et
(c) assemblage de l'élément de fermeture mécanique avec le substrat au moyen de l'élément adhésif;
**caractérisé en ce que** l'élément adhésif respectif est préparé de telle sorte qu'il ne recouvre pas complètement la surface de fixation arrière de l'élément de fermeture mécanique, la préparation de la multiplicité d'éléments de fermeture mécaniques s'effectuant avec à chaque fois un élément adhésif sur une surface de fixation arrière à l'étape b en séparant un élément de fermeture mécanique en plusieurs éléments de fermeture mécaniques et en appliquant à chaque fois un élément adhésif sur la surface de fixation arrière de chacun des éléments de fermeture mécaniques résultants.

10. Procédé selon la revendication 9, **caractérisé en ce que** l'élément adhésif est préparé de telle sorte qu'il couvre entre 60 % et 90 %, de préférence entre 70 % et 80 %, de la surface de fixation arrière de l'élément de fermeture mécanique.

11. Procédé selon la revendication 9 ou 10, **caractérisé en ce que** les étapes a. à c. s'effectuent en continu.

12. Dispositif de fabrication d'une bande de fermeture de couche-culotte selon l'une quelconque des revendications 1 à 8, comprenant : une alimentation en substrat pour préparer un substrat; une alimentation en élément de fermeture pour préparer au moins un élément de fermeture mécanique avec une surface de mise en prise avant et avec une surface de fixation arrière ; un dispositif de revêtement pour appliquer un adhésif sur la surface de fixation arrière de l'au moins un élément de fermeture mécanique ; et un dispositif de contact pour assembler l'au moins un élément de fermeture mécanique avec le substrat au moyen de l'adhésif, **caractérisé en ce que** le dispositif de revêtement comprend un dispositif de buse avec une multiplicité d'orifices de buse pour préparer une multiplicité correspondante d'éléments adhésifs et **en ce que** le dispositif de revêtement comprend en plus un accessoire de guidage pour guider l'au moins un élément de fermeture mécanique, lequel accessoire de guidage comprend une multiplicité d'évidements pour guider une multiplicité d'éléments de fermeture mécaniques, un orifice de buse correspondant dans le dispositif de buse étant associé à chaque évidement de l'accessoire de guidage et chacun des orifices de buse ayant un diamètre en direction transversale qui est plus petit que le diamètre de l'évidement correspondant dans l'accessoire de guidage en direction transversale.

13. Dispositif selon la revendication 12, **caractérisé en ce que** l'accessoire de guidage et le dispositif de buses sont réalisés de manière intégrée, de préférence sous la forme d'un module avec une multiplicité d'évidements en forme de T.

14. Couche-culotte, comprenant une ou plusieurs bandes de fermeture de couche-culotte selon l'une quelconque des revendications 1 à 8.
